(19) Europäisches Patentamt
European Patent Office
Office européen des brevets

(11) Publication number: **0 024 727**
**B1**

(12) **EUROPEAN PATENT SPECIFICATION**

(45) Date of publication of patent specification: **26.05.82**

(51) Int. Cl.³: **C 07 H 15/24,**
A 61 K 31/70 //C07H13/08

(21) Application number: **80105099.8**

(22) Date of filing: **27.08.80**

(54) **Anthracycline glycosides, processes for their preparation and pharmaceutical composition containing them.**

(30) Priority: **01.09.79 GB 7930392**

(43) Date of publication of application:
**11.03.81 Bulletin 81/10**

(45) Publication of the grant of the patent:
**26.05.82 Bulletin 82/21**

(84) Designated Contracting States:
**AT BE CH DE FR GB LI NL SE**

(56) References cited:
**FR - A - 2 439 792**

**JOURNAL OF MEDICINAL CHEMISTRY, vol. 22, no. 2 February 1979 CASSINELLI et al. Synthesis and antitumor activity of 4′-O-methyldaunorubicin, 4′-O-methyladriamycin, and their 4′-epi analogues. pages 121—123.**

(73) Proprietor: **FARMITALIA CARLO ERBA S.p.A.**
**Via Carlo Imbonati n.24**
**I-20159 Milan (IT)**

(72) Inventor: **Bargiotti, Alberto**
**Via Donati n.8**
**Milan (IT)**
Inventor: **Cassinelli, Giuseppe**
**Via G. Matteotti 13**
**Voghera (PV) (IT)**
Inventor: **Penco, Sergio**
**Via Crimea n.13**
**I-20147 Milano (IT)**
Inventor: **Arcamone, Federico**
**Via 4 Novembre 26**
**Nerviano (MI) (IT)**
Inventor: **di Marco, Aurelio Dr.**
**Via Plinio 59**
**Milan (IT)**

(74) Representative: **Hansen, Bernd, Dr.rer.nat. et al,**
**Hoffmann . Eitle & Partner Patentanwälte**
**Arabellastrasse 4**
**D-8000 München 81 (DE)**

EP 0 024 727 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Anthracycline glycosides, processes for their preparation and pharmaceutical composition containing them

The invention relates to anthracycline glycosides, process for their preparation and to pharmaceutical compositions containing them. Also included in the invention are new branched-chain amino-deoxy sugar derivatives used in the preparation of said anthracycline glycosides.

The invention provides anthracycline glycosides of the general formula I:

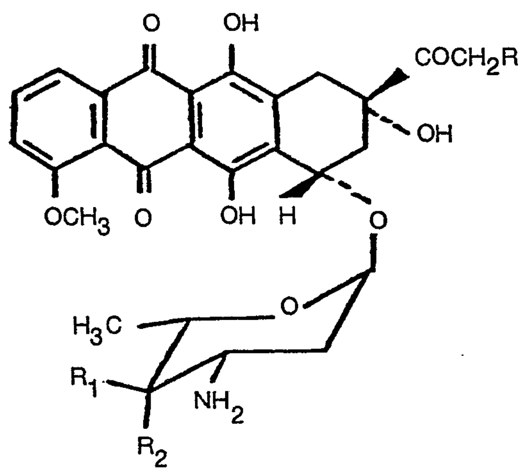

I

in which R represents a hydrogen atom or a hydroxy group, one of $R_1$ and $R_2$ represents a methyl group, and the other of $R_1$ and $R_2$ represents a hydroxy group, and their pharmaceutically acceptable acid addition salts. The anthracycline glycosides I may be named as follows:

| | | |
|---|---|---|
| $R = H, R_1 = CH_3, R_2 = OH$ | : | 4′-C-methyl-daunorubicin |
| $R = R_2 = OH, R_1 = CH_3$ | : | 4′-C-methyl-doxorubicin |
| $R = H; R_1 = OH; R_2 = CH_3$ | : | 4′-epi-4′-C-methyl-daunorubicin |
| $R = R_1 = OH, R_2 = CH_3$ | : | 4′-epi-4′-C-methyl-doxorubicin |

They are referred to hereinafter, for convenience, as compounds I—A, I—B, I—C and I—D respectively.

The compunds according to the invention may be prepared according to a process characterized in that daunomycinone is condensed with 2,3,6-trideoxy-4-C-methyl-4-O-p.nitrobenzoyl-3-trifluoro-acetamido-L-lyxo-hexopyranosyl chloride or 2,3,6-trideoxy-4-C-methyl-4-O-p.nitrobenzoyl-L-arabino-hexopyranosyl chloride, the protecting groups are then removed from the resultant protected glycoside derivative in a manner known per se, the so obtained free glycosidic base (R = H) of general formula I is then isolated, optionally in the form of a pharmaceutically acceptable acid addition salt, and is then optionally brominated at the 14-position followed by hydrolysis and isolation to yield the corresponding free glycosidic base (R = OH) of general formula I, optionally in the form of a pharmaceutically acceptable acid addition salt.

This process will be described in more detail hereinafter with reference to specific embodiments.

Compounds I—A and I—C may be prepared by a process, which process comprises condensing daunomycinone with 2,3,6-trideoxy-4-C-methyl-4-O-p.nitrobenzoyl-3-trifluoroacetamido-L-lyxo-hexo-pyranosyl chloride or 2,3,6-trideoxy-4-C-methyl-4-O-p.nitrobenzoyl-L-arabino-hexopyranosyl chloride (hereinafter, for convenience, referred to as compounds II—A and II—B respectively) in an inert organic solvent, in the presence of a soluble silver salt catalyst, and of a molecular sieve as dehydrating agent, and removing the N-trifluoroacetyl protecting group from the resultant anthracylcine glycoside by mild alkaline hydrolysis.

The mild alkaline hydrolysis may be carried out in accordance with the method described in United States Patent Specification No. 4,112,076. The inert organic solvent is suitably methylene dichloride or chloroform. Silver trifluoromethanesulphonate is the preferred silver salt. Compounds I—A and I—C may be isolated as their hydrochlorides.

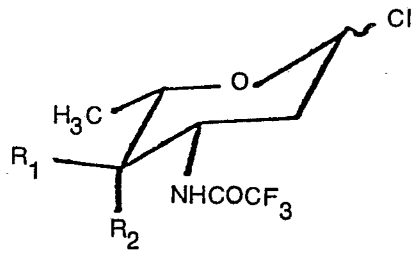

II

II—A : $R_1$ = $CH_3$, $R_3$ = 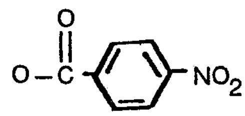

II—B : $R_1$ = 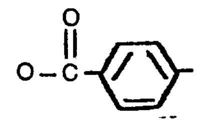, $R_2$ = $CH_3$

Compounds I—B and I—D may be prepared from compounds I—A and I—C respectively by a further process within the scope of the invention. The said further process comprises brominating the compound I—A or I—C at the 14-position and hydrolysing the resultant 14-bromoderivative to give the compound I—B or I—D. The bromination and hydrolysis conditions may be those described in British Patent Specification No. 1,217,133 or those described in United States Patent Specification No. 3,803,124.

Compounds II—A and II—B are themselves novel. They may be respectively prepared from methyl 2,3,6-trideoxy-4-C-methyl-3-trifluoroacetamido-α-L-lyxo-hexopyranoside (IV—A) and from methyl 2,3,6-trideoxy-4-C-methyl-3-trifluoroacetamido-α-L-arabinohexopyranoside (IV—B), known compounds described in British Patent Application No. 41,937/78, filed on October 25, 1978 (equivalent to FR - 2439792).

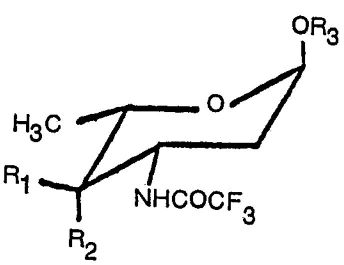

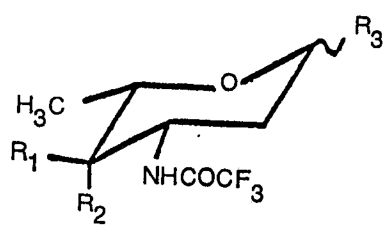

IV—A : $R_1$ = $R_3$ = $CH_3$, $R_2$ = OH

IV—B : $R_1$ = OH, $R_2$ = $R_3$ = $CH_3$

V—A : $R_1$ = $CH_3$, $R_2$ = OH, $R_3$ = H

V—B : $R_1$ = OH, $R_2$ = $CH_3$, $R_3$ = H

VI—A : $R_1$ = $CH_3$, $R_2$ = $R_3$ = 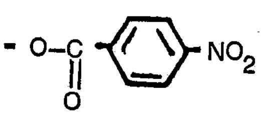

VI—B : $R_1$ = $R_3$ = 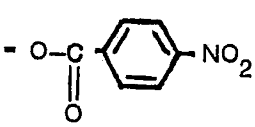, $R_2$ $CH_3$

Acid hydrolysis of compunds IV—A and IV—B gives respectively 2,3,6-trideoxy-4-C-methyl-α-L-lyxo-hexopyranose (V—A) and α-L-arabino-hexopyranose (V—B) which by treatment with p-nitrobenzoyl chloride in methylene dichloride and triethylamine in the presence of a catalytic amount of 4-dimethylaminopyridine are transformed into the corresponding 1,4-di-O-p.nitrobenzoyl derivatives VI—A and VI—B. Treatment of these with dry hydrogen chloride in anhydrous methylene dichloride affords respectively compounds II—A and II—B.

Compounds I—A, I—B, I—C and I—D are useful as antitumour agents and compounds II—A and II—B are useful as intermediates for the preparation of compounds I—A, I—B, I—C and I—D.

Daunomycinone is described and claimed in British Patent Specification No. 1,003,383.

The invention further provides a pharmaceutical composition comprising an anthracycline glycoside of the general formula I or a pharmaceutically acceptable addition salt thereof in admixture with a therapeutically acceptable diluent or carrier.

## BIOLOGICAL ACTIVITY DATA

### *Antitumor activity*

The new compounds have been tested on HeLa cells *in vitro* (time exposure to the drugs: 24 hours) and on P—388 ascitic leukemia in mice in comparison with daunorubicin (daunomycin) and doxorubicin (adriamycin).

The results of the *in vitro* tests are shown in Table 1.

TABLE 1 – Effect on HeLa cells cloning efficiency *in vitro* (a)

| COMPOUND | $ID_{50}$ (ng/ml) |
|---|---|
| Daunorubicin . HCl | 9 |
| 4'-C-methyl-daunorubicin . HCl (I–A) | 35 |
| 4'-epi-4'-C-methyl-daunorubicin . HCl (I–C) | 2.8 |
| Doxorubicin . HCl | 8.4 |
| 4'-C-methyl-doxorubicin . HCl (I–B) | 18 |
| 4'-epi-4'-C-methyl-doxorubicin . HCl (I–D) | 0.62 |

a) HeLa cells were exposed to the drugs for 24 hours, then plated. Colonies number was evaluated 5 days later.

The *in vitro* data obtained in mice are reported in Table 2.

All the new compounds at the tolerated dose showed on P—388 leukemia an activity comparable to or higher than that of the parent compounds.

TABLE 2 – Activity on ascitic P–388 leukemia in mice.

| Compound | Dose (a) mg/Kg | T/C (b) % | Toxic (c) deaths | LTS (d) |
|---|---|---|---|---|
| Daunorubicin . HCl | 2.9<br>4.4 | 175<br>180 | 0/40<br>3/39 | |
| 4'-C-methyl-daunorubicin . HCl (I–A) | 10<br>20 | 150<br>155 | 0/10<br>0/10 | |
| 4'-epi-4'-C-methyl-daunorubicin . HCl (I–C) | 0,29<br>0,44<br>0,66 | 140<br>163<br>158 | 0/10<br>0/10<br>1/20 | |
| Doxorubicin . HCl | 6,6<br>10 | 193<br>227 | 0/30<br>1/28 | 2/30<br>5/28 |
| 4'-C-methyl-doxorubicin . HCl (I–B) | 6<br>7,7<br>10 | 186<br>172<br>233 | 0/10<br>0/10<br>2/20 | <br><br>3/20 |
| 4'-epi-4'-C-methyl-doxorubicin . HCl (I–D) | 1<br>2 | 150<br>156 | 0/10<br>0/10 | |

a) Mice were treated i.p. on day 1 after tumor cell inoculation.

b) Median survival time of treated mice/median survival time of control mice × 100.

c) Evaluated on the base of macroscopic autoptic findings.

d) Long-time survival.

The invention is illustrated by the following Examples in which degrees of temperature are degrees Celcius.

Example 1

*4-O-p.nitrobenzoyl-2,3,6-trideoxy-4-C-methyl-3-trifluroacetamido-L-lyxo-hexopyranosyl chloride (II—A)*

A solution of 1g (3.7 mmoles) of methyl 2,3,6-trideoxy-4-C-methyl-3-trifluoroacetamido-$\alpha$-L-lyxo-hexopyranoside (IV—A) in 20 ml of acetic acid and 80 ml of water was reacted at 100° for 2 hours. The solution was evaporated and 2,3,6-trideoxy-4-C-methyl-3-trifluoroacetamido-$\alpha$-L-lyxo-hexopyranose (V—A; 0.95 g, 95%) was obtained as a white solid; m.p. 181—182°, $[\alpha]_D^{23°} = -127°$ (c = 1.0 in methanol). The PMR spectrum ($CDCl_3$ + DMSO $-$ $d_6$) showed inter alia absorptions at: 1.05 (s, $CH_3$—C—4), 1.16 (d, $CH_3$—C—5), and 5.27 $\delta$ (C—1—H). A solution of 0.75 g (2.9 mmoles) of compound V—A in a mixture of 10 ml of triethylamine and 20 ml of dry methylene dichloride was treated under stirring with 2.20 g of *p*.nitrobenzoyl chloride and 0.220 g of 4-dimethylaminopyridine, then heated at 45° for 90 minutes. The solution was evaporated off and the residue was dissolved in 100 ml of chloroform, washed with 10% sodium bicarbonate solution and with water, and then the chloroform solution was dried over sodium sulphate. The residue obtained by evaporation was chromatographed on a silica gel column. Elution with a 95:5 chloroform: acetone mixture gave 1,4-di-O-*p*.nitrobenzoyl-2,3,6-trideoxy-4-C-methyl-3-trifluoroacetamido-L-lyxo-hexopyranose (VI—A, 1.55 g, 95%): m.p. 168—170°, $[\alpha]_D^{23°} = -35°$ (c=1 in chloroform).

A solution of 1.10 g (2.0 mmoles) of compound VI—A in 25 ml of dry methylene dichloride was saturated at 0° with anhydrous hydrogen chloride.

The resulting precipitate of *p*.nitrobenzoic acid was filtered off under anhydrous conditions and the filtrate was evaporated to give 2,3,6-trideoxy-4-C-methyl-4-O-*p*.nitrobenzoyl-3-trifluoroacetamido-L-lyxo-hexo-pyranosyl chloride (II—A, 0.80 g) as a white solid suitable for the subsequent coupling reaction without further purification.

Example 2

*4-O-p.nitrobenzoyl-2,3-6-trideoxy-4-C-methyl-3-trifluoroacetamido-L-arabino-hexopyranosyl chloride (II—B)*

Acid hydrolysis of methyl 2,3,6-trideoxy-4-C-methyl-3-trifluoroacetamido-$\alpha$-L-arabino-hexopyranoside (IV—B, 0.80 g, 3 mmoles) as described in Example 1 gave 2,3,6-trideoxy-4-C-methyl-3-trifluoroacetamido-$\alpha$-L-arabino-hexopyranose (V—B, 0.700 g, 90%) as a solid; m.p. 110—111°, $[\alpha]_D^{23°} = -83°$ (c=1 in methanol). The PMR spectrum ($CDCl_3^D$ + DMSO-$d_6$) showed inter alia adsorptions at: 1.10 (s, $CH_3$—C—4), 1.17 (d, $CH_3$—C—5), and 5.23 $\delta$ (broad s, C—1—H).

Treatment of compound V—B (0.60 g, 2.33 mmoles) with *p*-nitrobenzoyl chloride as described in Example 1 gave the corresponding 1,4-di-O-*p*.nitrobenzoyl derivative (VI—B, 1.030 g, 80%): m.p. 151°, $[\alpha]_D^{23°} = -21°$ (c= 1.1 in chloroform).

A solution of 0.90 g (1.62 mmoles) of compound VI—B in 20 ml of dry methylene dichloride was saturated at 0° with anhydrous hydrogen chloride. After filtering off the precipitated *p*.nitrobenzoic acid, the solution was evaporated to dryness to give 2,3,6-trideoxy-4-C-methyl-4-O-*p*.nitrobenzoyl-3-trifluoroacetamido-L-arabino-hexopyransoyl chloride (II—B, 0.650 g) as a white solid suitable for the subsequent coupling reaction without further purification.

Example 3

*4'-C-Methyl-daunorubicin (I—A) (IMI 105)*

To a solution of daunomycinone (0.900 g, 2.26 mmoles) in dry methylene dichloride (90 ml) was added 2,3,6-trideoxy-4-C-methyl-4-O-*p*.nitrobenzoyl-3-trifluoroacetamido-L-lyxo-hexopyranosyl chloride (II—A, 0.800 g, prepared as described in Example I) in 15 ml of methylene dichloride and molecular sieve (4 Å Merck, 6 g). The mixture was then treated with 0.58 g of silver trifluoromethanesulphonate in anhydrous diethyl ether (15 ml) under vigorous stirring. After 1 hour at room temperature, the reaction mixture was neutralized with a saturated aqueous solution of sodium bicarbonate and the organic phase was separated off and evaporated under vacuum. Chromatographic purification of the crude residue on a silica gel column, using 95:5 chloroform: acetone as eluent, gave (0.965 g, 65%) 4'-C-methyl-4'-O-*p*.nitrobenzoyl-N-trifluoroacetyl-daunorubicin, m.p. 172—173°, $[\alpha]_D^{23°} = +420°$ (c = 0.05 in chloroform).

The PMR spectrum ($CDCl_3$) showed inter alia absorption at: 1.28 (d, $CH_3$—C—5'), 1.56 (s, $CH_3$—C—4'), 2.48 (s, $CH_3CO$), 4.02 (s, $OCH_3$), 5.21 (broad s, C—7—H), 5.50 (broad s, C—1'—H), 13.11 and 13.92 $\delta$ (two s, phenoic OH).

A solution of 0.8 g of the compound prepared above in 15 ml of acetone was treated with 45 ml of 0.1 N aqueous sodium hydroxide and stirred under nitrogen at room temperature. After 1 hour the reaction mixture was adjusted to pH 3.5 with 1 N aqueous hydrogen chloride and then extracted with chloroform to eliminate impurities. The aqueous phase, adjusted to pH 8.0, was extracted twice with chloroform. The combined organic extracts were dried over sodium sulphate, concentrated to a small volume and acidified to pH 4.5 with 0.25 N methanolic hydrogen chloride. Addition of an excess of

diethyl ether gave 4'-C-methyl-daunorubicin (I—A) as the hydrochloride (0.515 g 88%), m.p. 162—163° (with decomposition), $[\alpha]_D^{23°} = +320°$ (c = 0.05 in methanol).

Example 4

*4'-C-Methyl-doxorubicin (I—B) (IMI 109)*

A solution of 4'-C-methyl-daunorubicin hydrochloride (I—A, 0.450 g, 0.78 mmoles) prepared as described in Example 3, in a mixture of 6 ml of anhydrous methanol, 17.5 ml of dioxan and 0.45 ml of ethyl orthoformate was treated with 1.8 ml of a solution containing 0.95 g of bromine in 10 ml of chloroform. After 1.5 hours at 10° the reaction mixture was poured into a mixture of 90 ml of diethyl ether and 45 ml of petroleum ether. The resultant red precipitate, after being filtered off and washed with diethyl ether, was dissolved in a mixture of 15 ml of acetone and 15 ml of 0.25 N aqueous hydrogen bromide. After 20 hours at 30° the reaction mixture was treated with 0.68 g of sodium formate in 7.5 ml of water and stirred at 30° for 48 hours. The reaction mixture was extracted with chloroform in order to remove some lipophilic impurities. The aqueous phase, after being adjusted to pH 7.6 with aqueous sodium bicarbonate, was repeatedly extracted with chloroform until the extracts were colourless. The combined chloroform extracts were dried on sodium sulphate and evaporated to a small volume (about 20 ml) under vacuum to the resulting red solution, adjusted to pH 3.5 with anhydrous methanolic hydrogen chloride, was added diethyl ether in excess to give 4'-C-methyl-doxorubicin (I—B, 0.410 g) as the hydrochloride, m.p. 185—186°C (with decomposition), $[\alpha]_D^{23°} = +227°$ (c = 0.05 in methanol).

Example 5

*4'-Epi-4'-C-methyl-daunorubicin (I—C) (IMI 110)*

The synthesis of the title compound, starting from daunomycinone and 2,3,6-trideoxy-4-C-methyl-4-O-*p*.nitrobenzoyl-3-trifluoroacetamido-L-arabino-hexopyranosyl chloride (II—B), prepared as described in Example 2, was carried out according to the procedure described in Example 3.

4'-Epi-4'-C-methyl-daunorubicin (I—C) was obtained as the hydrochloride in the form of red crystals, m.p. 187—188° (with decomposition), $[\alpha]_D^{23°} = +285°$ (c = 0.05 in methanol).

Example 6

*4'-Epi-4'-C-methyl-doxorubicin (ID)*

The compound I—C, prepared as described in Example 5, was transformed into the corresponding 14-hydroxy derivative, according to the procedure described in Example 4. 4'-Epi-4'-C-methyl-doxorubicin (I—D) was obtained as the hydrochloride in the form of red crystals, m.p. 169—170° (with decomposition), $[\alpha]_D^{23°} = +250°$ (c = 0.04 in methanol).

**Claims**

1. Anthracycline glycosides of the general formula I:

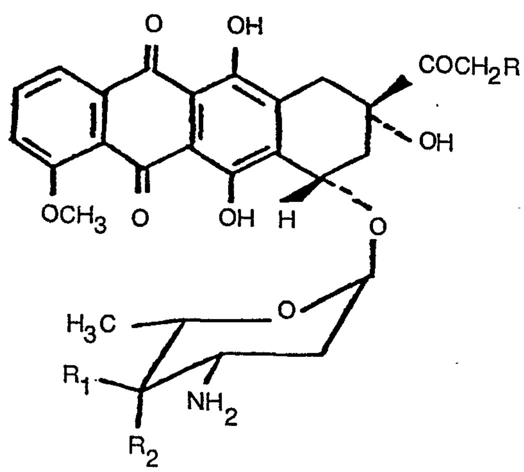

I

in which R represents a hydrogen atom or hydroxy group, one of $R_1$ and $R_2$ represents a methyl group, and the other of $R_1$ and $R_2$ represents a hydroxy group, and their pharmaceutically acceptable acid addition salts.

2. 4'-C-methyl-daunorubicin.
3. 4'-C-methyl-doxorubicin.
4. 4'-epi-4'-C-methyl-daunorubicin.
5. 4'-epi-4'-C-methyl-doxorubicin.
6. A process for the preparation of anthracycline glycosides according to claim 1, characterized in

that daunomycinone is condensed with 2,3,6-trideoxy-4-C-methyl-4-O-p.nitrobenzoyl-3-trifluoro-acetamido-L-lyxo-hexopyranosyl chloride or 2,3,6-trideoxy-4-C-methyl-4-O-p.nitrobenzoyl-L-arabino-hexopyranosyl chloride, all protecting groups are then removed from the resultant protected glycoside derivatives in a manner known per se, the so obtained free glycosidic base (R = H) of general formula I is then isolated, optionally in the form of a pharmaceutically acceptable acid addition salt, and is then optionally brominated at the 14-position followed by hydrolysis and isolation to yield the corresponding free glycosidic base (R = OH) of general formula I, optionally in the form of a pharmaceutically acceptable acid addition salt.

7. A process according to claim 6, wherein the protecting groups are removed by mild alkaline hydrolysis.

8. A process according to claim 6, wherein bromination is carried out with a solution of bromine in an organic solvent.

9. A process according to claim 6, wherein hydrolysis of the 14-bromo derivatives is carried out with an aqueous solution of sodium formate.

10. A pharmaceutical composition comprising a compound of general formula I, according to claim 1, or a pharmaceutically acceptable salt thereof in admixture with a pharmaceutically acceptable diluent or carrier.

**Revendications**

1. Glycosides d'anthracycline de formule générale I:

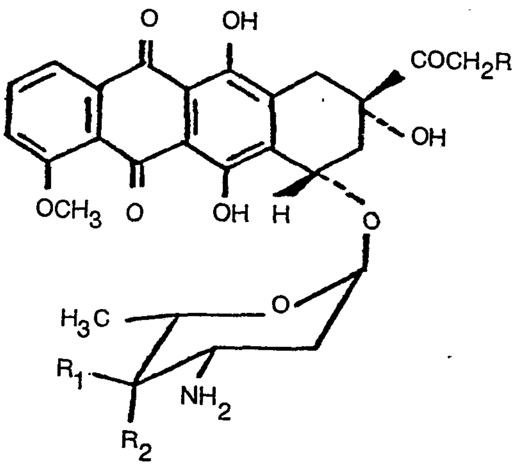

I

dans laquelle R représente un atome d'hydrogène ou un groupe hydroxyle, l'un des $R_1$ et $R_2$ représente un groupe méthyle, et l'autre des $R_1$ ou $R_2$ représente un groupe hydroxyle, et leurs sels acides d'addition pharmaceutiquement acceptables.

2. La 4'-C-méthyl-daunorubicine.

3. La 4'-C-méthyl-doxorubicine.

4. La 4'-épi-4'-C-méthyl-daunorubicine.

5. La 4'-épi-4'-C-méthyl-doxorubicine.

6. Procédé pour la préparation des glycosides d'anthracycline selon la revendication 1, caractérisé par le fait que: la daunomycinone est condensée avec le chlorure de 2,3,6-tridésoxy-4-C-méthyl-4-O-p.nitrobenzoyl-3-triluoroacétamido-L-lyxo-hexapyranosyle ou le chlorure de 2,3,6-tridésoxy-4-C-méthyl-4-O-p.nitrobenzoyl-L-arabino-hexopyranosyle, qu'ensuite tous les groupes protecteurs du dérivé glycosidique protégé résultant sont éliminés de manière connue en soi, que la base glycosidique libre ainsi obtenue (R=H) de formule générale I est ensuite isolée, éventuellement sous la forme d'un sel acide d'addition pharmaceutiquement acceptable, puis qu'elle est éventuellement bromée sur la position 14, puis hydrolysée et isolée pour obtenir la base glycosidique libre correspondante (R=OH) de formule générale I, éventuellement sous la forme d'un sel acide d'addition pharmceutiquement acceptable.

7. Procédé selon la revendication 6, dans lequel les groupes protecteurs sont éliminés par hydrolyse alcaline ménagée.

8. Procédé selon la revendication 6, dans lequel la bromation est réalisée avec une solution de brome dans un solvant organique.

9. Procédé selon la revendication 6, dans lequel l'hydrolyse des dérivés 14-bromo est réalisée avec une solution aqueuse de formiate de sodium.

10. Composition pharmaceutique contenant un composé de formule générale I, selon la revendication 1, ou un sel pharmaceutiquement acceptable de ce composé, en mélange avec un diluant ou un véhiculeur pharmaceutiquement acceptable.

**Patentansprüche**

1. Anthracyclinglykoside de allgemeinen Formel I:

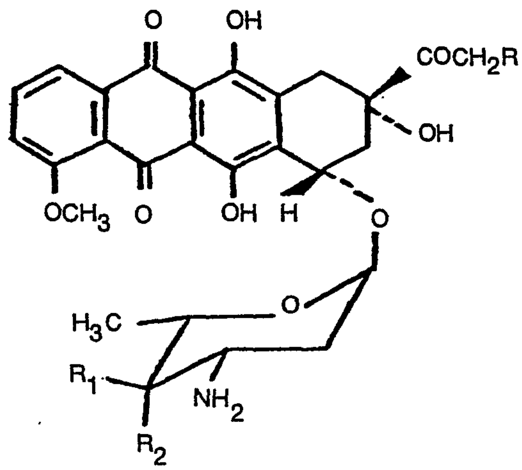

I

worin R ein Wasserstoffatom oder eine Hydroxygruppe, ein $R_1$ und $R_2$ eine Methylgruppe und das andere $R_1$ und $R_2$ eine Hydroxygruppe bedeuten sowie deren pharmazeutisch annehmbaren Säureadditionssalze.

2. 4'-C-Methyl-daunorubicin.

3. 4'-C-Methyl-doxorubicin.

4. 4'-Epi-4'-C-methyl-daunorubicin.

5. 4'-Epi-4'-C-methyl-doxorubicin.

6. Verfahren zur Herstellung von Anthracyclinglykosiden gemäß Anspruch 1, dadurch gekennzeichnet, daß man Daunomycinon mit 2,3,6-Trideoxy-4-C-methyl-4-O-para-nitrobenzoyl-3-trifluoroacetamido-L-lyxo-hexopyranosyl-chlorid oder 2,3,6-Trideoxy-4-C-methyl-4-O-para-nitrobenzoyl-L-arabino-hexopyranosylchlorid kondensiert, von dem erhaltenen geschützten Glykosidderivat alle Schutzgruppen in bekannter Weise entfernt, die so erhaltene freie Glykosidbase (R=H) der allgemeinen Formel I dann osiliert, gewünschtenfalls in Form eines pharmazeutisch annehmbaren Säureadditionssalzes, und dann gewünschtenfalls in der 14-Stellung bromiert und anschließlich hydrolysiert und isoliert unter Ausbildung der entsprechenden freien Glykosidbase (R=OH) der allgemeinen Formel I, gewünschtenfalls in Form eines pharmazeutisch annehmbaren Säureadditionssalzes.

7. Verfahren gemäß Anspruch 6, dadurch gekennzeichnet, daß man die Schutzgruppen durch milde alkalische Hydrolyse entfernt.

8. Verfahren gemäß Anspruch 6, dadurch gekennzeichnet, daß man die Bromierung mit einer Lösung von Brom in einem organischen Lösungsmittel vornimmt.

9. Verfahren gemäß Anspruch 6, dadurch gekennzeichnet, daß man die Hydrolyse des 14-Bromoderivats mit einer wäßrigen Lösung von Natriumformiat durchführt.

10. Pharmazeutische Zusammensetzung enthaltend eine Verbindung der allgemeinen Formel I gemäß Anspruch 1 oder pharmazeutisch annehmbares Salz davon im Gemisch mit einem pharmazeutisch annehmbaren Verdünnungsmittel oder Träger.